# FASCICULE DE BREVET EUROPEEN

(11) **EP 2 875 795 B1**
(45) Date de publication et mention de la délivrance du brevet: **04.05.2016**
(21) Numéro de dépôt: 14189157.2
(22) Date de dépôt: 16.10.2014
(51) Int. Cl.: A61F 2/32, A61F 2/36, A61F 2/46, A61F 2/30

(54) **Procédé de conditionnement d'une tête d'articulation prothétique et d'une pièce articulaire formant une cavité d'articulation destinée à recevoir cette tête d'articulation**
Verfahren zum Ausrichten eines Prothesengelenkkopfes und eines Gelenkteils, das eine Gelenkpfanne zur Aufnahme dieses Gelenkkopfes bildet
Method for packaging a head of a prosthetic joint and a joint part forming a joint cavity intended for receiving said joint head

(30) Priorité: 19.11.2013 FR 1361366
(43) Date de publication de la demande: 27.05.2015
(73) Titulaire: Groupe Lepine, 69730 Genay (FR)
(72) Inventeur: Amorim, Cédric, 69580 Sathonay Camp (FR)
(74) Mandataire: Jeannet, Olivier

(56) Documents cités:
- US-A- 4 100 626
- US-A- 4 457 306
- US-A- 4 676 798
- US-A1- 2009 048 682
- US-A1- 2009 281 631

## Description

La présente invention concerne un procédé de conditionnement d'une tête d'articulation prothétique de forme sensiblement sphérique et d'une pièce articulaire formant une cavité d'articulation destinée à recevoir cette tête d'articulation. La tête d'articulation prothétique peut notamment être celle d'une prothèse de hanche et ladite pièce articulaire peut notamment être le noyau de glissement que comprend un implant cotyloïdien.

Pour éviter, ou tout au moins réduire au maximum, le risque de luxation d'une prothèse totale de la hanche, il est nécessaire d'aménager, sur le noyau de l'implant cotyloïdien, un collet à l'entrée de la cavité d'articulation, ayant un diamètre légèrement inférieur à celui de l'équateur de la tête fémorale, et d'insérer cette tête en force au-delà de ce collet, jusque dans cette cavité, en profitant de la légère possibilité de déformation élastique du polyéthylène constituant le noyau. Cette opération est réalisée au cours de l'intervention chirurgicale, par le chirurgien ou son assistant, au moyen d'un ancillaire ayant un pas de vis, permettant de visser la tête en force dans le noyau cotyloïdien afin de permettre à cette tête de franchir ledit collet.

Cette technique a cependant pour inconvénient important d'engendrer des rayures dans la paroi délimitant la cavité d'assemblage que présente la tête d'articulation pour son assemblage à la tige fémorale, voire même des fissures dans l'épaisseur de cette tête, compte tenu de l'effort circulaire et non constant exercé sur la tête au cours de l'insertion. Ces détériorations causées à la tête d'articulation ne sont pas sans incidences sur la solidité de la liaison de la tête à la tige fémorale et donc sur la pérennité de la prothèse.

La présente invention a pour objectif de remédier à cet inconvénient important.

La publication de brevet américain N° US 4,457,306 décrit une pince à deux branches reliées de façon pivotante l'une à l'autre, dont une est conformée de manière à être apte à prendre appui contre une tête d'articulation de prothèse de hanche et dont l'autre est conformée de manière à être apte à prendre appui contre une pièce articulaire de prothèse de hanche, cette pince permettant la mise en place de la tête d'articulation dans la cavité d'articulation que forme ladite pièce articulaire.

La pièce articulaire selon ce document ne comprend pas de collet tel que précité et la pince décrite permet uniquement un engagement sans forcer de la tête d'articulation dans la cavité d'articulation. Cette pince n'est donc pas en mesure de remédier à l'inconvénient précité.

Le procédé selon l'invention comprend les étapes consistant à :
- utiliser une machine pour insérer la tête dans la cavité d'articulation selon l'axe de cette cavité, sans mouvement circulaire de cette tête au moment de cette insertion, cette machine incluant un poinçon déplaçable selon un axe, comportant la tête d'articulation à son extrémité libre, et un support de montage fixe de la pièce articulaire, ce support se faisant de telle sorte que l'axe de cette cavité coïncide avec l'axe de déplacement du poinçon ;
- utiliser une pièce de calage de la tête d'articulation dans la cavité d'articulation, comprenant une partie de montage apte à être insérée et retenue dans la cavité d'assemblage que comprend cette tête pour son assemblage à une pièce d'ancrage osseux, et une partie de calage prolongeant ladite partie de montage, dimensionnée pour faire saillie de cette cavité d'assemblage après l'insertion de la partie de montage dans cette cavité d'assemblage ;
- une fois la tête d'articulation insérée dans ladite cavité d'articulation, mettre en place ladite pièce de calage dans ladite cavité d'assemblage de manière à réaliser le calage de la tête d'articulation par rapport à la pièce formant la cavité d'articulation.

L'inventeur a pu observer que, si l'assemblage d'une tête d'articulation à une pièce articulaire se fait par le praticien au cours de l'intervention chirurgicale, c'est du fait qu'il pourrait se produire un basculement de la tête d'articulation dans la cavité d'articulation au cours du transport de l'ensemble tête-pièce articulaire depuis l'emplacement de fabrication vers l'emplacement d'utilisation. Un tel basculement serait particulièrement problématique compte-tenu de la difficulté à faire pivoter la tête dans la cavité articulaire eu égard aux frottements pouvant exister entre cette tête et la pièce articulaire, et du fait de l'absence de prise efficace sur la tête. Il en résulterait des manipulations difficiles, en cours d'intervention, et des risques de contamination des pièces à implanter.

L'inventeur a ainsi pu concevoir de réaliser l'assemblage tête-pièce articulaire en un lieu privilégié, en particulier chez le fabricant de cette tête et de cette pièce articulaire, et à l'aide d'une machine adaptée, réalisant l'insertion de la tête dans la cavité d'articulation selon l'axe de cette cavité, sans mouvement circulaire de cette tête au moment de cette insertion, donc sans risque de causer des dommages à la tête lors de cette insertion, mais ceci à condition de caler la tête par rapport à la pièce articulaire une fois l'insertion réalisée, au moyen de ladite pièce de calage. L'ensemble est ensuite stérilisé et placé dans un emballage de transport.

Au moment de l'assemblage de la tête d'articulation à la pièce d'ancrage osseux permettant d'ancrer cette tête à l'os concerné, en particulier une tige médullaire fémorale, l'orifice de ladite cavité d'assemblage se trouve positionné de façon que cet assemblage puisse être réalisé dans les meilleures conditions.

De préférence, la partie de montage présente une forme correspondant à celle de ladite cavité d'assemblage, mais de dimensions légèrement supérieures à celles de cette cavité, de telle sorte que la pièce de calage soit apte à être retenue dans cette cavité par des frottements surmontables manuellement ; le procédé comprend alors l'étape consistant à retirer manuellement la pièce de calage hors de la cavité d'assemblage.

La mise en place et le retrait de cette pièce de calage sont ainsi réalisés de manière particulièrement simple et rapide.

De préférence, ladite partie de calage présente des dimensions telles qu'elle est apte à être saisie manuellement.

Cette partie de calage permet ainsi une saisie et une manipulation faciles de l'ensemble tête-pièce articulaire. Elle permet, le cas échéant de déplacer la tête d'articulation dans la cavité de la façon la plus approprié à l'assemblage à réaliser.

De préférence, dans ce cas, cette partie de calage présente une forme aplatie, en palette, favorisant la préhension manuelle de l'ensemble tête-pièce articulaire.

La pièce de calage peut comporter des informations relatives à l'ensemble tête-pièce articulaire auquel elle est reliée, facilitant ainsi l'identification de cet ensemble.

Le procédé selon l'invention comprend avantageusement les étapes consistant à placer l'ensemble tête-noyau-pièce de calage dans un sachet hermétique puis à opérer un vide d'air dans ce sachet de telle sorte que le sachet s'applique étroitement sur ledit ensemble, et à sceller le sachet dans cet état de vide.

L'invention sera bien comprise, et d'autres caractéristiques et avantages de celle-ci apparaîtront, en référence au dessin schématique annexé, représentant, à titre d'exemple non limitatif, une forme de réalisation préférée d'une pièce de calage concernée.

La figure 1 est une vue en coupe axiale d'une tête d'articulation prothétique fémorale et du noyau d'un implant cotyloïdien formant une cavité d'articulation destinée à recevoir cette tête ;

la figure 2 est une vue de la tête et du noyau similaire à la figure 1, la tête étant montée sur un poinçon et le noyau étant immobilisé par un support de montage fixe ;

la figure 3 est une vue de la tête et du noyau similaire à la figure 1, la tête étant engagée dans la cavité d'articulation et une pièce de calage étant prête à être montée sur l'ensemble ;

la figure 4 est une vue de la tête et du noyau similaire à la figure 3, la pièce de calage étant montée sur l'ensemble tête-noyau ;

la figure 5 est une vue de la tête et du noyau similaire à la figure 4, l'ensemble tête-noyau-pièce de calage étant placé dans un sachet mis sous vide et scellé de manière étanche ; et

la figure 6 est une vue de la tête et du noyau similaire à la figure 4, montrant le calage opéré par ladite pièce de calage.

Les figures représentent la tête d'articulation prothétique 1 d'une prothèse de hanche et le noyau en polyéthylène 2 faisant partie d'un implant cotyloïdien.

La tête 1 est sphérique et comprend une cavité d'assemblage 3 en forme de cône à faible pente, cette cavité 3 permettant l'assemblage de la tête 1 à une pièce d'ancrage fémoral, en particulier au col d'une tige médullaire fémorale (non représentée). Cette tête 1 peut être métallique ou en céramique.

Le noyau 2 forme une cavité d'articulation 4 destinée à recevoir la tête 1 et comprend un collet 5 à l'entrée de cette cavité 4, ayant un diamètre légèrement inférieur à celui de l'équateur de la tête 1, ainsi qu'un cône d'entrée 6. La tête 1 est destinée à être insérée en force au-delà du collet 5, jusque dans la cavité 4. Dans l'exemple représenté, non limitatif, le noyau 2 forme, en dehors du cône d'entrée 6, une face extérieure 2a sphérique, de telle sorte que ce noyau puisse se mouvoir dans la coque métallique (non représentée) de l'implant cotyloïdien, ce dernier étant ainsi un implant de type dit "à double mobilité".

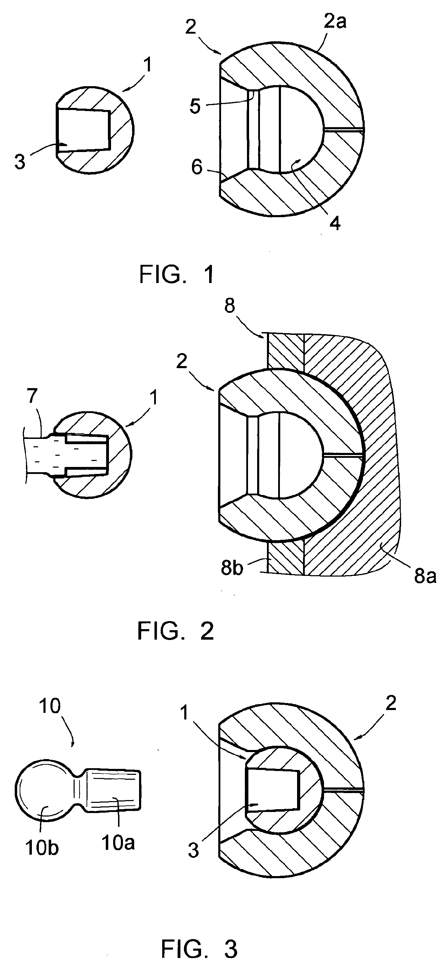

La figure 2 montre qu'une machine est utilisée pour insérer la tête 1 dans la cavité 4. Cette machine inclut un poinçon 7 sur l'extrémité libre duquel est montée la tête 1, et un support 8 de montage fixe du noyau 2 de telle sorte que l'axe de la cavité 4 coïncide avec l'axe de déplacement du poinçon 7. Ce support 8 comprend une base 8a et une mâchoire 8b de maintien du noyau 2. L'insertion de la tête 1 dans la cavité 4 est réalisée au moyen du mouvement du poinçon 7 en direction de la cavité 4, selon l'axe de cette cavité, par une pression axiale de la tête 1 contre le collet 5, sans mouvement circulaire de cette tête au moment de cette insertion.

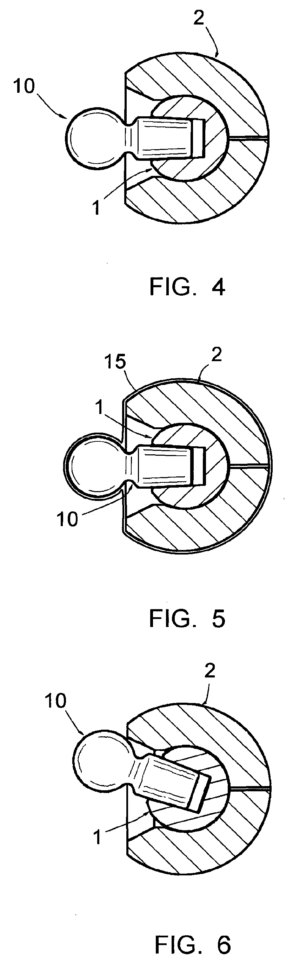

La tête 1, une fois insérée dans la cavité 4, est séparée du poinçon 7 et le noyau 2 est séparé du support 8, puis une pièce de calage 10 est mise en place dans la cavité d'assemblage 3, comme visible sur les figures 3 et 4. Cette pièce de calage 10 comprend une partie de montage 10a apte à être insérée et retenue dans la cavité d'assemblage 3 et une partie de calage 10b prolongeant cette partie de montage 10a de manière à ce que cette partie de calage 10b fasse saillie de cette cavité d'assemblage 3 après l'insertion de la partie de montage 10a dans la cavité d'assemblage 3, comme visible sur la figure 4.

La partie de montage 10a présente une forme de cône à faible pente correspondant à celle de la cavité 3, mais a des dimensions légèrement supérieures à celles de cette cavité, de telle sorte que la pièce de calage 10 soit apte à être retenue dans cette cavité 3 par des frottements surmontables manuellement. La pièce de calage 10 peut être en matière plastique courante, notamment en polypropylène.

La partie de calage 10b présente une base raccordée à la partie 10a et a une forme aplatie, en palette, et des dimensions telles qu'elle est apte à être saisie manuellement. Elle peut comporter des informations relatives à l'ensemble tête 1-noyau 2 auquel elle est reliée, facilitant ainsi l'identification de cet ensemble.

Une fois la pièce de calage 10 insérée dans la cavité 3, l'ensemble tête 1-noyau 2-pièce de calage 10 est placé dans un sachet hermétique 15 puis un vide d'air est opéré de telle sorte que ce sachet s'applique étroitement sur ledit ensemble, comme montré sur la figure 5. Le sachet 15 est scellé dans cet état de vide.

L'ensemble, dans cet état, peut être transporté depuis l'emplacement d'assemblage vers l'emplacement d'utilisation, sans risque de basculement de la tête 1 dans la cavité d'articulation 4.

Lors de l'opération chirurgicale, le sachet 15 est ouvert et retiré, et la partie 10b permet une manipulation aisée de l'ensemble tête 1-noyau 2. Si nécessaire, au moment de l'assemblage de la tête 1 à la pièce d'ancrage osseux permettant d'ancrer cette tête à l'os concerné, en particulier à la tige médullaire fémorale, la pièce de calage 10 peut être utilisée pour faire pivoter la tête 1 dans la cavité 4 jusqu'à la position la plus appropriée pour que cet assemblage puisse être réalisé dans les meilleures conditions, comme visible sur la figure 6. Une fois cette position obtenue, la pièce de calage 10 est retirée, par simple traction manuelle sur la partie 10b de celle-ci.

Ainsi que cela apparaît de ce qui précède, l'invention fournit un procédé de conditionnement permettant de réaliser l'assemblage tête 1 - noyau 2 (ou autre pièce articulaire) en un lieu privilégié, en particulier chez le fabricant de cette tête et de ce noyau, et à l'aide d'une machine adaptée, réalisant l'insertion de la tête 1 dans la cavité d'articulation 4 selon l'axe de cette cavité, sans mouvement circulaire de cette tête au moment de cette insertion, donc sans risque de causer des dommages à la tête 1 lors de cette insertion. La pièce de calage 10 permet non seulement d'empêcher tout risque de basculement de la tête 1 dans le noyau 2 lors du transport, mais également d'obtenir un positionnement optimal de la cavité d'assemblage 3 en vue de l'assemblage à ladite pièce d'ancrage osseux.

L'invention a été décrite ci-dessus en référence à une forme de réalisation fournie à titre d'exemple. Il va de soi qu'elle n'est pas limitée à cette forme de réalisation et qu'elle s'étend à toutes les autres formes de réalisation couvertes par les revendications annexées.

## Revendications

1. Procédé de conditionnement d'une tête d'articulation prothétique (1) de forme sensiblement sphérique et d'une pièce articulaire (2) formant une cavité d'articulation (4) destinée à recevoir cette tête d'articulation (1), la tête d'articulation (1) comprenant une cavité d'assemblage (3) pour son assemblage à une pièce d'ancrage osseux, et la pièce articulaire (2) comprenant un collet (5) à l'entrée de la cavité d'articulation (4), ayant un diamètre inférieur à celui de l'équateur de la tête d'articulation (1), cette tête d'articulation étant destinée à être insérée en force au-delà de ce collet (5), jusque dans ladite cavité d'articulation (4) ;
**caractérisé en ce qu'**il comprend les étapes consistant à :
- utiliser une machine pour insérer la tête d'articulation (1) dans la cavité d'articulation (4) selon l'axe de cette cavité, sans mouvement circulaire de cette tête au moment de cette insertion, cette machine incluant un poinçon (7) déplaçable selon un axe, comportant la tête d'articulation (1) à son extrémité libre, et un support (8) de montage fixe de la pièce articulaire (2), ce support se faisant de telle sorte que l'axe de cette cavité d'articulation (4) coïncide avec l'axe de déplacement du poinçon (7) ;
- utiliser une pièce (10) de calage de la tête d'articulation (1) dans la cavité d'articulation (4), comprenant une partie de montage (10a) apte à être insérée et retenue dans ladite cavité d'assemblage (3), et une partie de calage (10b) prolongeant la partie de montage (10a) et dimensionnée de manière à faire saillie de cette cavité d'assemblage (3) après l'insertion de la partie de montage (10a) dans cette cavité d'assemblage (3) ;
- une fois la tête d'articulation (1) insérée dans ladite cavité d'articulation (4), mettre en place ladite pièce de calage (10) dans ladite cavité d'assemblage (3) de manière à réaliser le calage de la tête d'articulation (1) par rapport à la pièce (2) formant la cavité d'articulation (4).

2. Procédé selon la revendication 1, **caractérisé en ce que** :
- ladite partie de montage (10a) présente une forme correspondant à celle de ladite cavité d'assemblage (3), mais de dimensions légèrement supérieures à celles de cette cavité, de telle sorte que la pièce de calage (10) soit apte à être retenue dans cette cavité (3) par des frottements surmontables manuellement ; et
- le procédé comprend l'étape consistant à retirer manuellement la pièce de calage (10) hors de la cavité d'assemblage (3).

3. Procédé selon la revendication 1 ou la revendication 2, **caractérisé en ce que** ladite partie de calage (10b) présente des dimensions telles qu'elle est apte à être saisie manuellement.

4. Procédé selon la revendication 3, **caractérisé en ce que** ladite partie de calage (10b) présente une forme aplatie, en palette, favorisant la préhension manuelle de l'ensemble tête-pièce articulaire (2).

5. Procédé selon l'une des revendications 1 à 4, **caractérisé en ce que** la pièce de calage (10) utilisée comporte des informations relatives à l'ensemble tête d'articulation (1)-pièce articulaire (2) auquel cette pièce est reliée.

6. Procédé selon l'une des revendications 1 à 4, **caractérisé en ce que** la tête d'articulation prothétique (1) est celle d'une prothèse de hanche et ladite pièce articulaire (2) est le noyau de glissement faisant partie de l'implant cotyloïdien que comprend cette prothèse.

7. Procédé selon l'une des revendications 1 à 6, **caractérisé en ce qu'**il comprend les étapes consistant à placer l'ensemble tête (1)-noyau (2)-pièce de calage (10) dans un sachet hermétique (15) puis à opérer un vide d'air dans ce sachet de telle sorte que le sachet (15) s'applique étroitement sur ledit ensemble, et à sceller le sachet (15) dans cet état de vide.

## Patentansprüche

1. Verfahren zur Verpackung einer prothetischen Gelenkkopf (1) von im Wesentlichen sphärische Form und einen Gelenkteil (2) einen Gelenkpfanne bilden (4), die für den Gelenkkopf (1) aufzunehmen bestimmt ist, der Gelenkkopf (1) mit einer Einschraubbohrung (3) für seine Montage auf einem Knochenverankerungsteil und das Gelenkteil (2) mit einem Kragen (5) am Eingang der Gelenkpfanne (4) mit einem Durchmesser kleiner als der Äquator der Gelenkkopf (1), der Gelenkkopf dazu bestimmt ist zwangsweise über den Kragen (5) in die Gelenkpfanne (4) eingesetzt werden;
**dadurch gekennzeichnet, dass** es die folgenden Schritte umfasst:
- Verwendung eine Maschine mit dem Gelenkkopf (1) in der Gelenkhöhle (4) entlang der Achse dieses Hohlraums eingefügt werden, ohne kreisförmige Bewegung des Kopfes zum Zeitpunkt dieser Insertion, diese Maschine mit einem Stempel (7), der entlang einer Achse mit dem Gelenkkopf (1) an seinem freien Ende, und einem Träger (8) zum festen Gelenkteil Halterung (2), dieser Träger derart ist, dass die Achse dieses Gelenkpfanne (4) mit der Verschiebeachse des Stempels (7) zusammenfällt;
- Verwendung, einen Keilteil (10) der Gelenkkopf (1) in den Gelenkpfanne (4) umfassend einen Montageabschnitt (10a) in dem Befestigungshohlraum (3) eingeführt und befestigt werden kann und ein Keilteil (10b) von dem Montageabschnitt (10a) erstrecken) und dimensioniert, um sich von dem Befestigungshohlraum (3) nach dem Einsetzen des Befestigungsabschnitts (10a) in dem Befestigungshohlraum (3) vorstehen;
- Sobald der Gelenkkopf (1) in die Gelenkpfanne (4) eingelegt ist, anordnen der Keilteil (10) in dem Befestigungshohlraum (3), um den Gelenkkopf (1) relativ zu dem Gelenkteil (2) den gemeinsamen Hohlraum bilden (4) zum Verkeilen.

2. Verfahren nach Anspruch 1, **dadurch gekennzeichnet, dass**:
- Der Montageabschnitt (10a) eine Form hat, die der entspricht das Befestigungshohlraum (3), aber Abmessungen etwas größer als diejenigen der Hohlraum, so daß der Keilteil (10) angepasst ist, in diesen Hohlraum (3) zurückgehalten werden durch manuell überwindbar Reibung; und
- Das Verfahren umfasst den Schritt den Keilteil (10) manuell Entfernen aus dem Befestigungshohlraum (3).

3. Verfahren nach Anspruch 1 oder Anspruch 2, **dadurch gekennzeichnet, daß** der Keilteil (10b) ist so dimensioniert, dass er angepasst ist, von Hand greifen werden.

4. Verfahren nach Anspruch 3, **dadurch gekennzeichnet, daß** der Keilteil (10b) eine abgeflachte Form hat, wie eine Palette, um manuelles Ergreifen die Gesamtheit Gelenkkopf - Gelenkteil (2) zu erleichtern.

5. Verfahren nach einem der Ansprüche 1 bis 4, **dadurch gekennzeichnet, dass** die Keilteil (10) verwendet wird, enthält Informationen zu der Gesamtheit Gelenkkopf (1) - Gelenkteil (2), an dem dieses Stück verbunden ist.

6. Verfahren nach einem der Ansprüche 1 bis 4, **dadurch gekennzeichnet, dass** die prothetische Gelenkkopf (1), dass einer Hüftprothese ist und das Gelenkteil (2) ist der gleitende Kernteil des Pfannenimplantat bildet, der dieser Prothese umfaßt.

7. Verfahren nach einem der Ansprüche 1 bis 6, **dadurch gekennzeichnet, daß** es umfasst die Schritte des Anordnens der Gesamtheit Gelenkkopf (1) - Kern (2) - Keilteil (10) in einem luftdichten Beutel (15) dann mit einem Luftvakuum in dieser Tasche zu betreiben, so dass der Beutel (15), die eng zusammen trifft und Versiegeln des Beutels (15) in diesem Vakuumzustand.

## Claims

1. Method for packaging a prosthetic joint head (1) of substantially spherical shape and a joint part (2) forming a joint cavity (4) intended for receiving the joint head (1), the joint head (1) comprising a mounting cavity (3) for its assembly on a bone anchoring part, and the joint part (2) comprising a collar (5) at the entrance to the joint cavity (4) having a diameter smaller than that of the equator of the joint head (1), the joint head being intended to be forcibly inserted beyond the collar (5), into said joint cavity (4);
**characterized in that** it comprises the steps of:
- using a machine to insert the joint head (1) in the joint cavity (4) along the axis of this cavity, without circular movement of the head at the time of this insertion, this machine including a punch (7) movable along an axis, with the joint head (1) at its free end, and a support (8) for fixedly mounting the joint part (2), this support being such that the axis of this joint cavity (4) coincides with the axis of displacement of the punch (7);
- using a wedge part (10) for wedging the joint head (1) in the joint cavity (4) comprising a mounting portion (10a) adapted to be inserted and retained in said mounting cavity (3), and a wedge portion (10b) extending from the mounting portion (10a) and dimensioned so as to protrude from the mounting cavity (3) after insertion of the mounting portion (10a) in said mounting cavity (3);
- once the joint head (1) is inserted into said joint cavity (4), placing said wedge part (10) in said mounting cavity (3) so as to wedge the joint head (1) relative to the joint part (2) forming the joint cavity (4).

2. Method according to claim 1, **characterized in that**:
- said mounting portion (10a) has a shape corresponding to that of said mounting cavity (3), but having dimensions slightly bigger than those of this cavity, so that the wedging part (10) is adapted to be retained in this cavity (3) by manually surmountable friction; and
- the method comprises the step of manually removing the wedging part (10) out of the mounting cavity (3).

3. Method according to claim 1 or claim 2, **characterized in that** said wedge portion (10b) has dimensions such that it is adapted to be manually grab.

4. Method according to claim 3, **characterized in that** said wedge portion (10b) is flattened-shaped, in the form of a pallet, facilitating manual gripping of the joint head-piece (2) assembly.

5. Method according to one of claims 1 to 4, **characterized in that** the wedging part (10) used contains information relating to the joint head (1)-joint part (2) assembly to which this part is connected.

6. Method according to one of claims 1 to 4, **characterized in that** the prosthetic joint head (1) is that of a hip prosthesis and said joint part (2) is the sliding core forming part of the acetabular implant that this prosthesis includes.

7. Method according to one of claims 1 to 6, **characterized in that** it comprises the steps of placing the head (1)-core (2)-wedging part (10) assembly in an airtight bag (15), then of making a vacuum in this bag such that the bag (15) closely applies on said assembly, and of sealing the bag (15) in this vacuum condition.
